# EUROPEAN PATENT SPECIFICATION

(11) **EP 4 338 131 B1**
(45) Date of publication and mention of the grant of the patent: **29.04.2026**
(21) Application number: 22808528.8
(22) Date of filing: 11.05.2022
(51) Int. Cl.: G16H 50/50, G16H 30/40, G16H 30/20, G06T 17/05, A61B 6/03

(54) **SYSTEMS AND METHODS FOR RECONSTRUCTING IMPLANTABLE DEVICE GEOMETRY FROM PATIENT-SPECIFIC IMAGING SCANS**
SYSTEME UND VERFAHREN ZUR REKONSTRUKTION DER GEOMETRIE EINER IMPLANTIERBAREN VORRICHTUNG AUS PATIENTENSPEZIFISCHEN BILDGEBUNGSSCANS
SYSTÈMES ET PROCÉDÉS DE RECONSTRUCTION DE GÉOMÉTRIE DE DISPOSITIF IMPLANTABLE À PARTIR D'EXAMENS D'IMAGERIE SPÉCIFIQUES À UN PATIENT

(30) Priority: 11.05.2021 US 202163187057 P
(43) Date of publication of application: 20.03.2024
(73) Proprietor: Ohio State Innovation Foundation, Columbus, OH 43210 (US); Georgia Tech Research Corporation, Atlanta, GA 30318 (US)
(72) Inventor: DASI, Lakshmi, Prasad, Dublin, OH 43017 (US); CHEN, Huang, Athens, GA 30605 (US); ESMAILIE, Fateme, Atlanta, GA 30309 (US); SAMAEE, Milad, Marietta, GA 30313 (US); SIVAKUMAR, Sri, Krishna, Dublin, Ohio 43016 (US); YEATS, Breandan, Andre, Butler, Atlanta, GA 30318 (US)
(74) Representative: Bryn Aarflot AS
(86) International application number: PCT/US2022/072250
(87) International publication number: WO 2022/241431

(56) References cited:
- WO-A1-2014/009294
- US-A1- 2017 143 494
- US-A1- 2019 298 450
- HOPF RAOUL ET AL: "Postoperative analysis of the mechanical interaction between stent and host tissue in patients after transcatheter aortic valve implantation", vol. 53, 28 February 2017 (2017-02-28), US, pages 15 - 21, XP093251593, ISSN: 0021-9290, Retrieved from the Internet <URL:https://pdf.sciencedirectassets.com/271132/1-s2.0-S0021929017X00031/1-s2.0-S0021929016313318/main.pdf?hash=9ad297fa879a51da0f2f3e87f3350aa5a27852d823803ef567451f26e11f7346&host=68042c943591013ac2b2430a89b270f6af2c76d8dfd086a07176afe7c76c2c61&pii=S0021929016313318&tid=spdf-63a2fcdb-6b74-4ffb-8e3f-3d6> DOI: 10.1016/j.jbiomech.2016.12.038
- GESSAT MICHAEL ET AL: "Image-Based Mechanical Analysis of Stent Deformation: Concept and Exemplary Implementation for Aortic Valve Stents", IEEE TRANSACTIONS ON BIOMEDICAL ENGINEERING, IEEE, USA, vol. 61, no. 1, 1 January 2014 (2014-01-01), pages 4 - 15, XP011534758, ISSN: 0018-9294, [retrieved on 20131219], DOI: 10.1109/TBME.2013.2273496
- MORGANTI S ET AL: "Simulation of transcatheter aortic valve implantation through patient-specific finite element analysis: Two clinical cases", JOURNAL OF BIOMECHANICS, PERGAMON PRESS, NEW YORK, NY, US, vol. 47, no. 11, 20 June 2014 (2014-06-20), pages 2547 - 2555, XP029015301, ISSN: 0021-9290, DOI: 10.1016/J.JBIOMECH.2014.06.007
- ZOU YUN, HAN QING, WENG XISHENG, ZOU YONGWEI, YANG YINGYING, ZHANG KESONG, YANG KERONG, XU XIAOLIN, WANG CHENYU, QIN YANGUO, WANG : "The precision and reliability evaluation of 3-dimensional printed damaged bone and prosthesis models by stereo lithography appearance", MEDICINE, WILLIAMS AND WILKINS, BALTIMORE., US, vol. 97, no. 6, 1 February 2018 (2018-02-01), US , XP093008135, ISSN: 0025-7974, DOI: 10.1097/MD.0000000000009797

## Description

### Priority

This application claims priority to U.S. Provisional Patent Application No. 63/187,057, filed May 11, 2021

### Background

Due to the inevitable structural deterioration of the bioprosthetic leaflets, patients underwent surgical aortic valve replacement (SAVR) or transcatheter aortic valve replacement (TAVR) may need a second intervention. Successful implantation of a TAV in a degenerated SAV or TAV has been reported in, e.g., as a minimally invasive solution to this problem. As the application of TAVR expanding to the median-risk and even low-risk population, there will be more patients who need a second or even a third TAVR. To avoid complications related to the ViV procedure, thorough pre-procedural planning must be carried out to address the valve anchoring, paravalvular leakage, and coronary obstructions associated with the existing valve.

Advanced pre-procedural planning usually involves modeling the valve deployment using finite element analyses (FEA) and assessing the hemodynamics using computational fluid dynamics (CFD) simulations. It is beneficial to determine the geometry of the existing valve accurately for high-fidelity pre-surgical simulations. However, the blooming artifacts caused by the metallic stent make the valve stent segmented from the patient's CT scan appear several times thicker. Furthermore, it is challenging to segment the bioprosthetic leaflets from CT scans due to a low signal strength of the tissue, making the prediction of coronary obstruction by the leaflets difficult. One way to address this issue is to use a PEA-based simulation that deploys the valve in the patient's pre-TAVR anatomy, trying to capture the final valve geometry. However, the errors associated with this method usually fall within 2-4mm. Attempts to reconstruct a clean stent frame have been carried out however, prior art methods are complicated and valve-specific or did not include leaflets and the skirt in the model.

Document HOPF RAOUL ET AL: "Postoperative analysis of the mechanical interaction between stent and host tissue in patients after transcatheter aortic valve implantation", JOURNAL OF BIOMECHANICS, vol. 53, 28 February 2017 (2017-02-28), pages 15-21 discloses the following: "The analysis is based on a finite element procedure to extract the contact forces between an implanted Nitinol stent and the surrounding host tissue using postoperative CT images. The methodology was applied for patients (N=46) which have undergone a TAVI procedure with the Medtronic Core Valve Revalving System (MCRS) to obtain corresponding deformation and force maps. The postoperative CT data were recorded for each patient in both systolic and diastolic phase of the heart cycle. (see abstract).

### Summary

An exemplary method and system are disclosed that uses an image-based registration framework to accurately recover the implanted device geometry (e.g., implanted TAV or SAV geometry) from patient-specific CT scans (e.g., in which the blooming artifacts are eliminated). The exemplary method and system are configured to reconstruct the patient-specific valve geometry from CT scans accurately by taking advantage of the already-known valve model and by adopting an image-based registration framework.

In some aspects, the exemplary system and method start by obtaining a clean model of an implanted device (e.g., SAV/TAV) from the device manufacturer or by reverse-engineering from micro-CT scans. Then, a few corresponding landmarks between this reference model (e.g., valve model) and a patient-specific implanted model (e.g., patient-specific stent) are used for initial passes of alignment. A subsequent intensity-based, nonrigid B-spline registration may be carried out, and the clean stent model may be modified to match that in the patient's CT scan accurately. Lastly, the transformations may be applied to the leaflets and skirt of the valve to generate the patient-specific valve model.

By only selecting a few landmarks manually, the recovered patient-specific implanted model (e.g., patient-specific valve model) can be generated that is highly accurate, e.g., with errors less than 0.5 mm.

The model may be used for post-surgical evaluations or in pre-procedural planning (e.g., ViV pre-procedural planning). The model can also be used for post-TAVR assessments and high-fidelity pre-surgical ViV evaluations. It can also be used as the ground truth for validating patient-specific valve deployment simulations.

The invention is defined in the independent claims 1 and 10.

### Brief Description of the Drawings

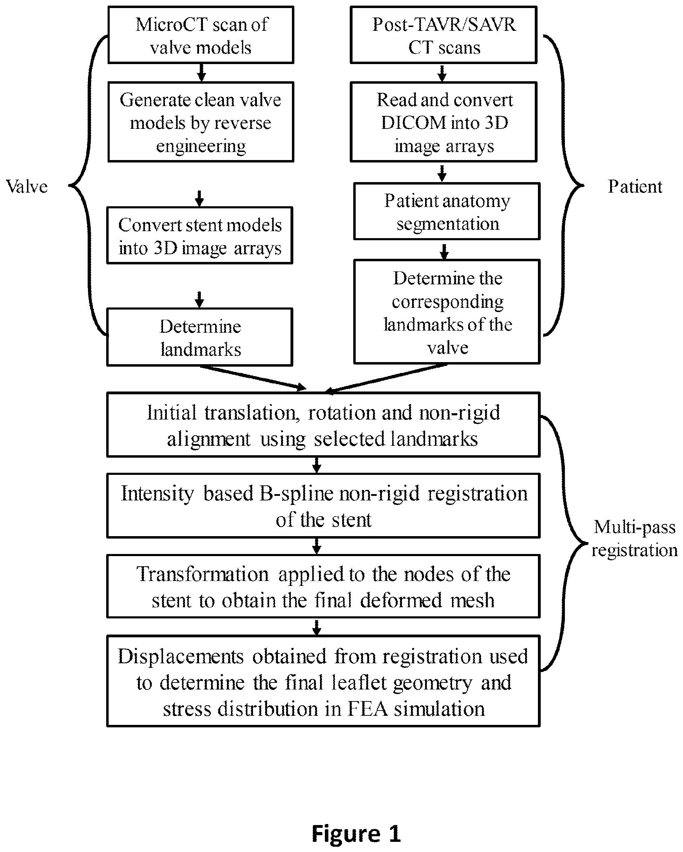

Figure 1 shows a flow chart of an image registration based method to reconstruct the TAV/SAV geometry from patient-specific CT scans.

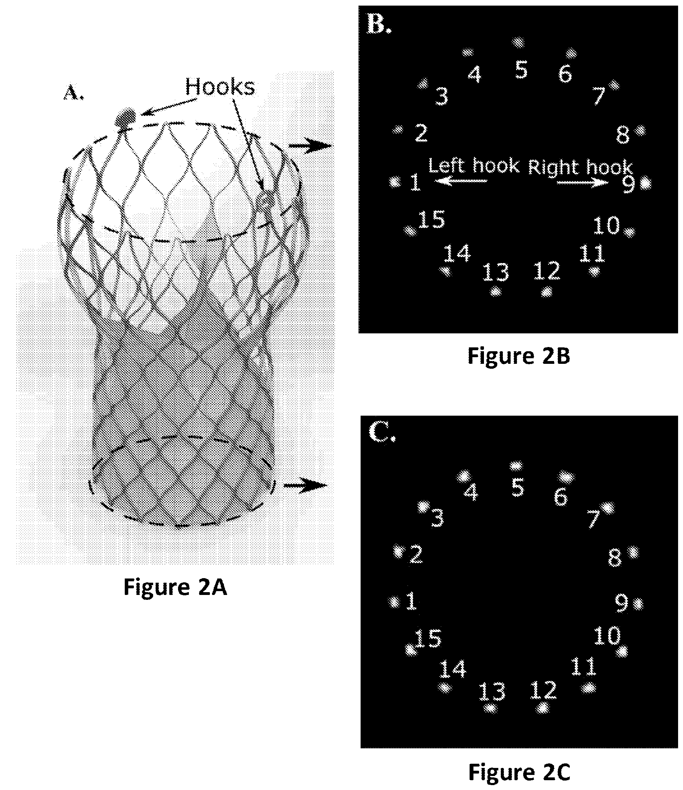

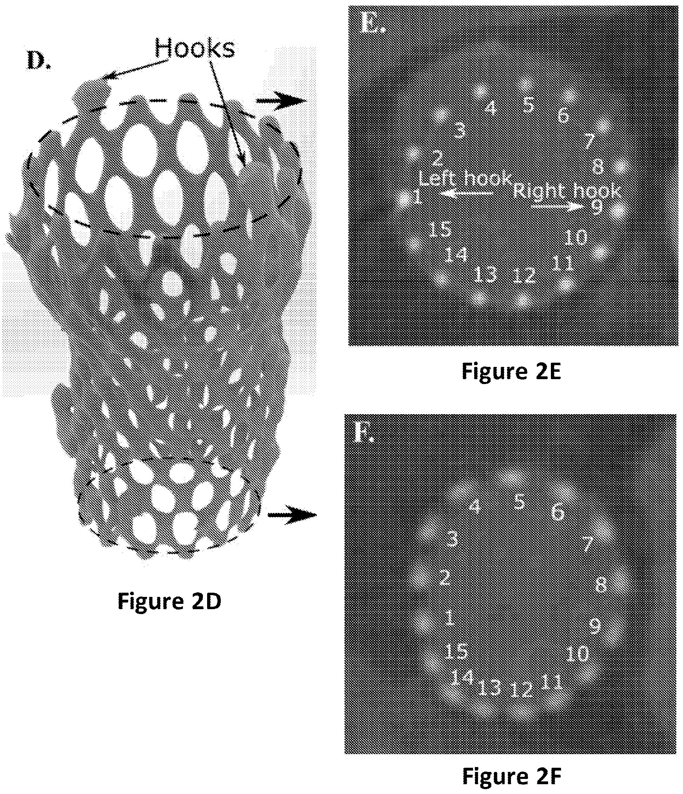

Figure 2 A shows a clean CAD model of a TAVR, obtained by reverse-engineering micro-CT scans. Figures 2B and 2C show section views of the voxelized model and selected landmarks. Figure 2D shows a patient-specific valve segmented by thresholding. Figures 2E and 2F show section views of the patient's CT scan, with the location and the landmarks corresponding to those in Figures 2B and 2D, respectively.

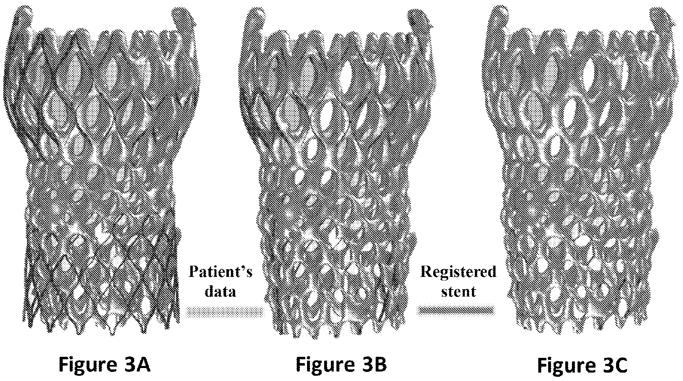

Figures 3A-3C show comparison between the stent model and patient's CT scan during different steps of the registration process. Figure 3A shows after an initial rigid alignment using provided landmarks, Figure 3B shows after a landmark-based non-rigid alignment, and Figure 3C shows after the final intensity-based B-spline deformable registration.

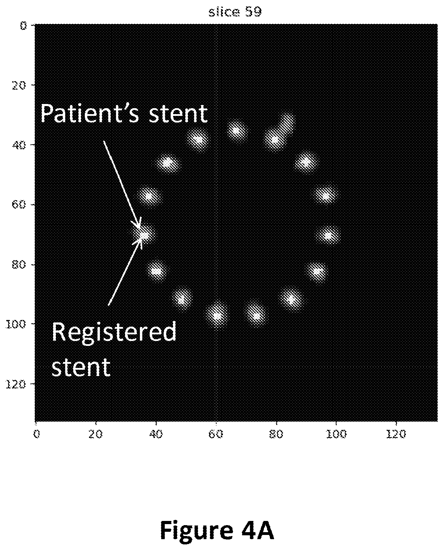

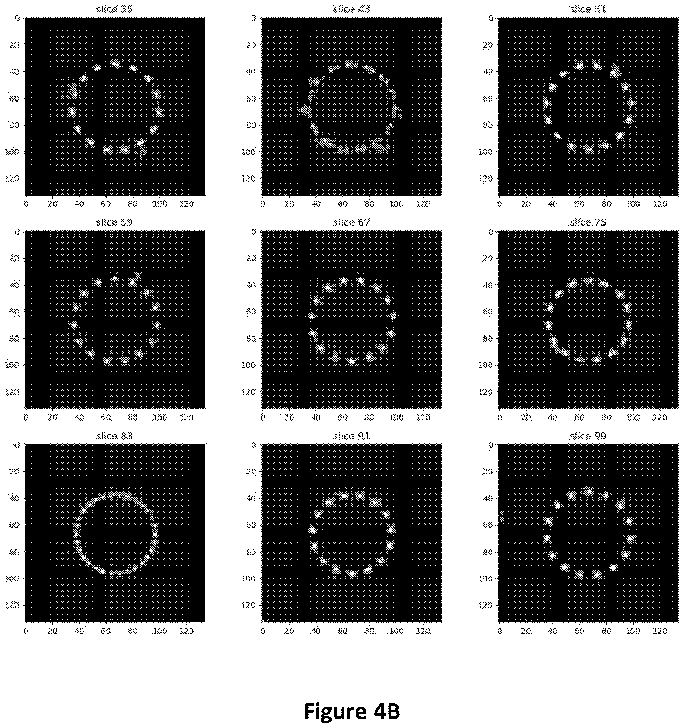

Figures 4A-4B show comparisons between the registered stent model and patient's CT scan in different section views. Figure 4A shows a sample annotated slice and Figure 4B shows slices at different locations

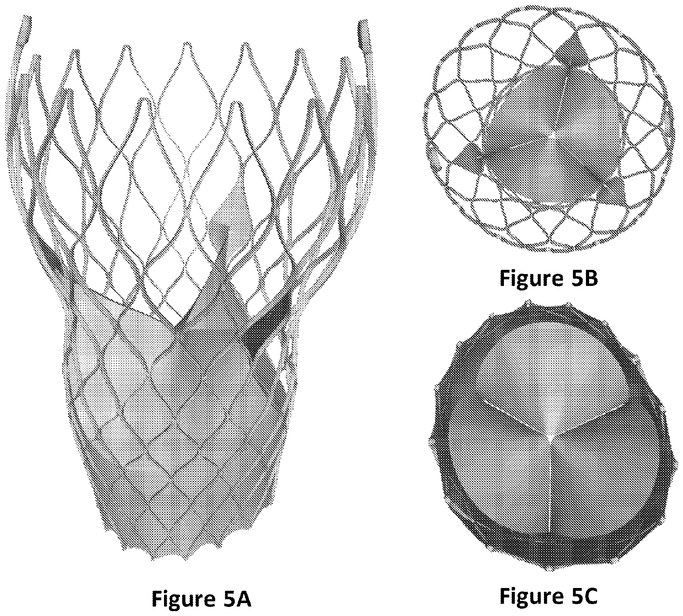

Figures 5A-5C show a sample patient-specific (self-expandable) valve model obtained using current method. Figures 5A, 5B and 5C correspond to side, top and bottom views of the model, respectively.

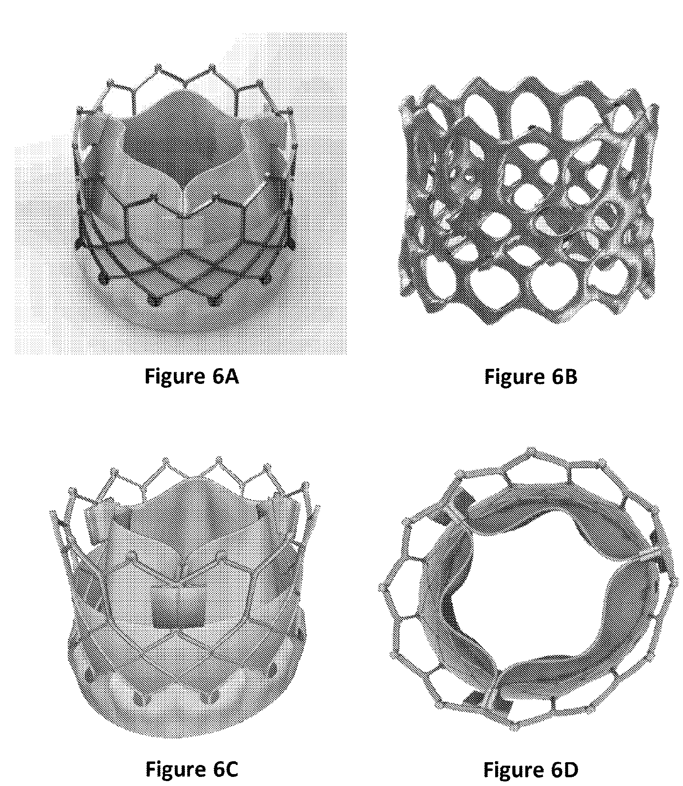

Figures 6A-6D show a sample patient-specific (balloon-expandable) valve model obtained using current method. Figure 6A shows the original model obtained from micro-CT scans, Figure 6B shows a comparison between the registered stent and the model segmented from patient's CT scans, and Figures 6C and 6D show the final registered valve model.

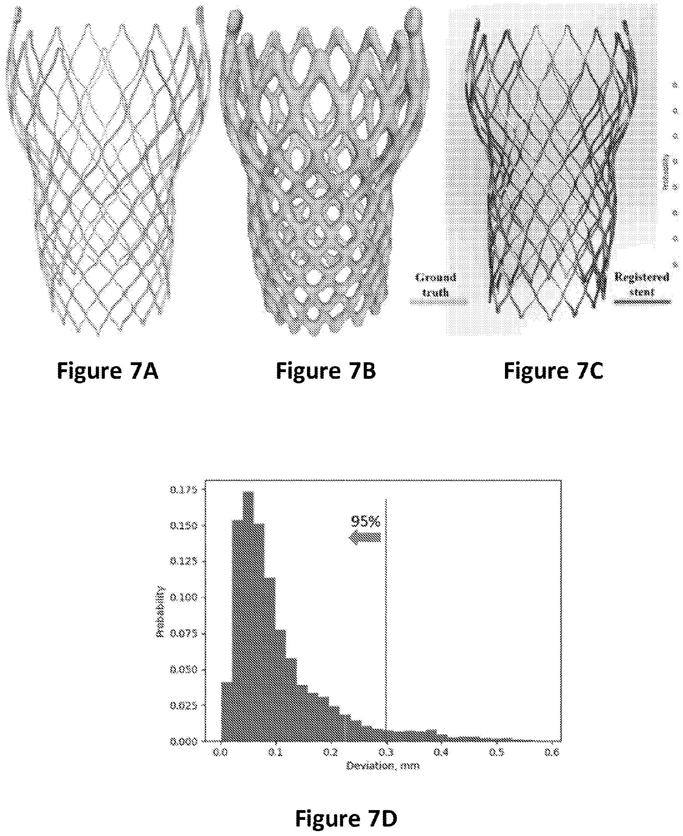

Figure 7 shows synthetic error assessment. Figure 7A shows a known stent after deployment - ground truth. Figure 7B shows a stent after applying a Gaussian blurring algorithm, mimicking the blooming artifact. Figure 7C shows comparisons between the registered stent and the ground truth. The registered stent is shown in darker grey. Figure 7D is a histogram showing the distribution of deviations (in mm) between the nodes.

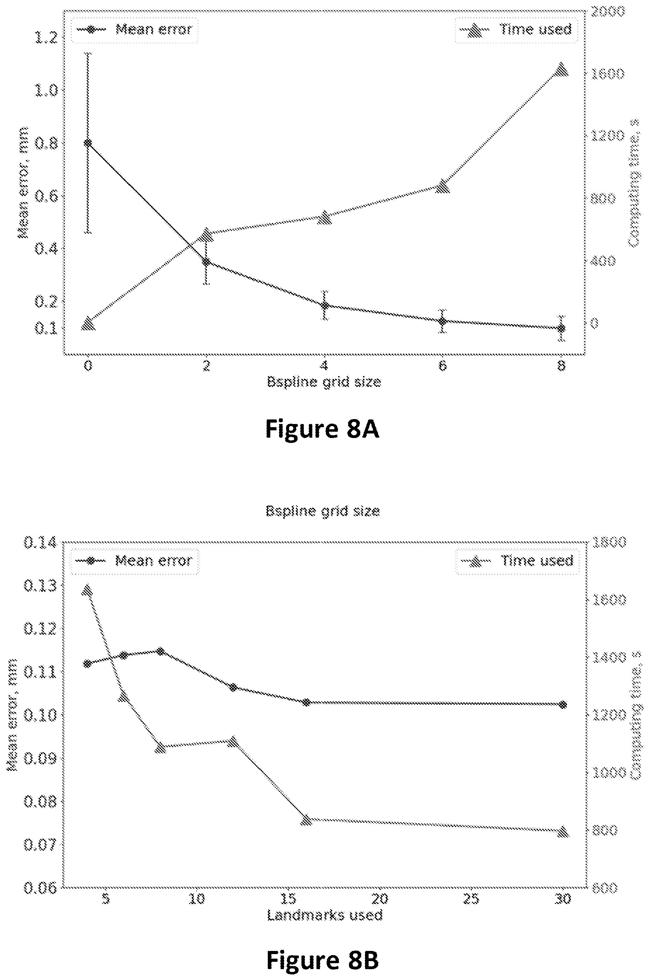

Figure 8 shows synthetic error assessment and sensitivity analysis. Figure 8A shows effects of the grid size on mean error (ten cases) and computing time (only one Evolut case). Figure 8B shows effects of the number of landmarks selected on registration accuracy and computing time for one Evolut case.

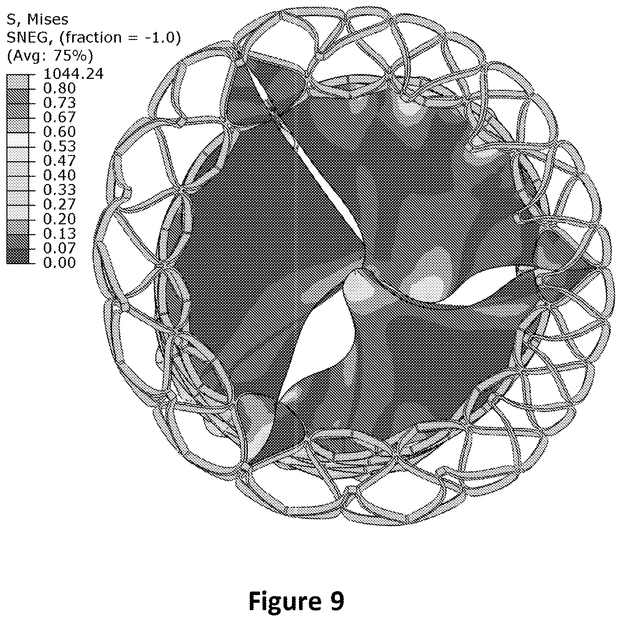

Figure 9 shows the recovered leaflet geometry and stress distribution using FEA simulations. Note the differences when compared with Figure 4B and 4C. Scale shows stress distribution in MPa.

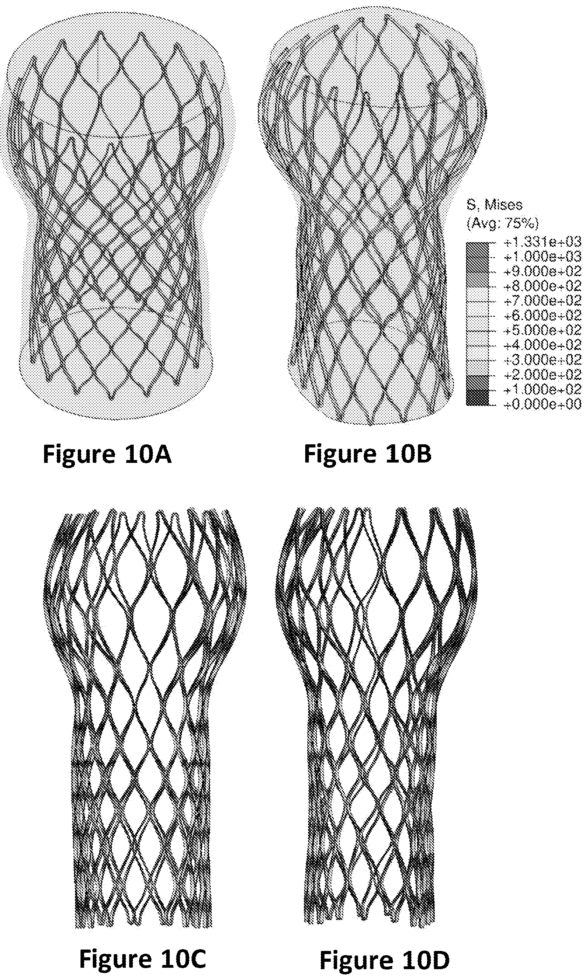

Figures 10A-10D show FEA simulations to estimate the stress distribution in the stent. Figure 10A shows a stent with undeformed bounding box; Figure 10B shows a stent crimped by the bounding box; Figures 10C shows estimated stress distribution compared with Figures 10D the ground truth. Scale shoes stress distributions in MPa.

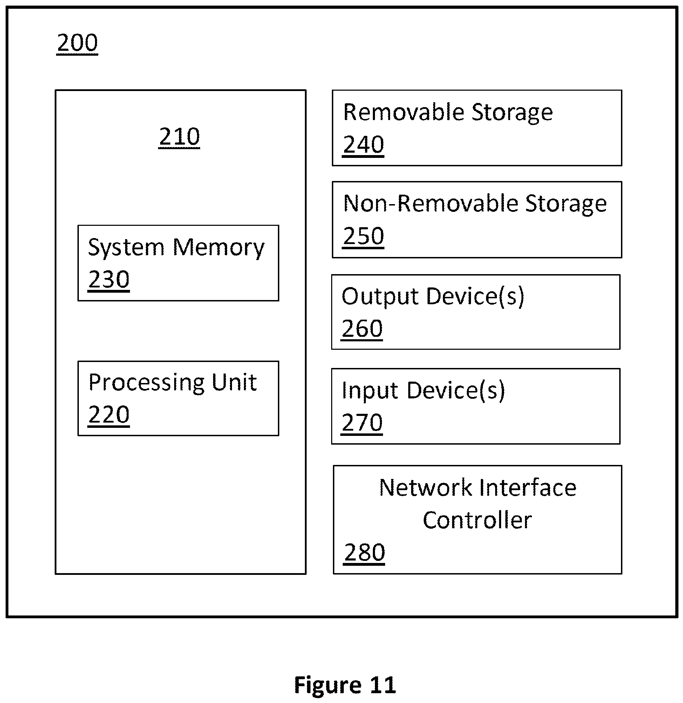

Figure 11 shows an illustrative computer architecture for a computer system capable of executing the software components that can use the output of the exemplary method described herein.

### Detailed Description

A system and fast and accurate method to reconstruct patient-specific valve geometries from CT scans. This approach takes advantage of the already-known valve topologies. Guided by a few manually selected landmarks, the known valve model is deformed to fit the patient-specific data in the intensity space using an image registration-based framework. Results have shown that the constructed patient-specific valve model is highly accurate, with mean errors around 0.1 mm, lower than the typical resolutions of cardiac CT scans. The shape and stress distributions of the deformed leaflets can be recovered in FEA simulations using the boundary conditions derived from the deforming stent. It is also possible to reasonably estimate the stress distributions in the stents using a simple crimping method. The reconstructed model can be used in high-fidelity pre-surgical ViV planning or post TAVR assessment. It can also serve as the ground truth for validating patient-specific valve deployment simulations. This method can also be used to reconstruct the geometries of other stented medical devices from CT

The flowchart describing the exemplary method is shown in Figure 1, which consists of three main parts: Obtaining a clean valve model and some easily identifiable landmarks, determining corresponding landmarks in the patient's CT scan, and applying the multi-pass registration algorithm.

Figure 1 shows a flow chart of an image registration based method to reconstruct an implant device geometry (e.g., TAV/SAV device geometry) from patient-specific CT scans.

In Figure 1, the exemplary system and method start by obtaining a clean model of an implanted device (e.g., SAV/TAV) from the device manufacturer or by reverse-engineering from micro-CT scans. Then, a few corresponding landmarks between this reference model (e.g., valve model) and a patient-specific implanted model (e.g., patient-specific stent) are used for initial passes of alignment. A subsequent intensity-based, nonrigid B-spline registration may be carried out, and the clean stent model may be modified to match that in the patient's CT scan accurately. Lastly, the transformations may be applied to the leaflets and skirt of the valve to generate the patient-specific valve model.

### Clean valve model and landmarks

Where a CAD (Computer-Aided Design) model from the manufacturer cannot be obtained, a high-quality valve model can be reconstructed by reverse-engineering the micro-CT scans of the valve. Typical resolutions of such scans are well below 50µm. Contrast agents can be sprayed onto the leaflets and skirt to enhance their signal strength. The valve model segmented from the scans can be readily used in registration and modeling. However, a clean CAD model is desired when high mesh quality is required. To obtain such models, one can use various software to reverse-engineering the scanned data into defect-free CAD models. As an example, Figure 2A shows a reconstructed Evolut valve model. The resolution of the scan was 50µm and SolidWorks was used to build the model.

The following step is to determine some easily identifiable landmarks of the valve model. For example, the Evolut valve shown in Figure 2A has two prominent hooks on the top of the stent, which are used in crimping. Guided by these two hooks, one could quickly identify all the 15 strut tips on the top of the stent, labeled clockwise from 1-15 (dashed line in Figure 2A and Figure 2B). In addition, the 15 strut tips at the bottom could also be easily identified (Figure 2C). The absolute coordinates of the 30 landmarks were recorded in the model's coordinate system. This step only needs to be carried out once for one type of valve, as long as the valve topology is unchanged. The landmarks can be hand-picked by a clinician or can be obtained by artificial intelligence (A.I.) and/or machining learning approaches. The number and location of the landmarks are not limited to the described ones.

### Patient's valve model and landmarks

The DICOM files from the patient's cardiac CT scans may be read, cropped around the valve, and resampled into a 3D array. A simple thresholding algorithm may then be applied to segment the stent. As shown in Figure 2D, the stent model was obtained using a threshold of 1000 HU (Hounsfield Unit) from a patient's post-TAVR CT scan. Note that the stent wireframe is about 5-10 times thicker than the clean model shown in Figure 2A. Following the same procedure, one can find all the corresponding 30 landmarks in this patient's valve model (Figures 2E and 2F). The coordinates of the landmarks were recorded in the 3D array's coordinate system. Generally, it is not necessary to use all the 30 landmarks in most cases. As shown by the following example, a total of 10 landmarks (5 on top and 5 at the bottom) works very well. The landmarks can be hand-picked by a clinician or can be obtained by A.I. and/or machining learning approaches. The number and location of the landmarks are not limited to the described ones.

### Multi-pass registration framework:

The exemplary multi-pass registration framework may be based on the open-source Insight Toolkit platform and is implemented in Python (Python Software Foundation, Wilmington, DE). Before registration, the stent of the clean valve model may be voxelized and converted into a 3D array. The size of this array may match with that of the patient's cropped array around the valve.

In an example, the array size was employed of 133x182x134 with a voxel spacing of 0.35 mm. Two sample slices of the voxelized array are shown in Figures 2B and 2C. To start, these two models were roughly aligned using only 10 landmarks as discussed before (top: 1, 4, 7, 9, 12; bottom: 1, 4, 7, 10, 13, Figure 2). The initial alignment consisted of scaling, rigid rotation, and translation. Figure 3 shows a comparison between a stent model and a patient's CT scan during different steps of the registration process of the method of Figure 1.

The initial result is shown in Figure 3A, where the clean stent roughly matches the patient's model. Subsequently, the second pass of deformable B-spline transformation was used to improve the initial alignment (Figure 3B). The method works by dividing the 3D domain into a mesh of control points with uniform spacings. By matching the corresponding landmarks, a deformation field controlled by B-splines is computed and applied to the whole 3D volume. In this example, a 4x4x4 mesh grid was used. Although the two models' alignment is already good when compared in Figure 3B, there are still discrepancies. A final intensity-based B-spline registration step corrected all the misalignment and produced a highly accurate result (Figure 3C). In this step, the 3D domain was divided into a 6x6x6 mesh grid and its deformation was controlled by cubic B-splines. Mutual information of the joint histogram was used as the similarity measure, and the line search gradient descent algorithm was adopted as the optimizer.

Other similarity measures (cross-correlation) and optimizers can also be used to achieve similar results. The registration method for the initial alignments is not limited to the method described here, and the number of passes and the order of implementation can be modified. Also, the registration method for the final step is not limited to B-spline registration described here. Other non-rigid registration methods can be used as well without changing the framework.

To verify the accuracy of this method, in Figure 4, the registered stent is overlaid with slices from the patient's CT data. The darker blurred blobs are stents that appeared in the CT scans, and the bright spots are from the voxelized registered clean stent. Figure 4 shows comparisons between a registered stent model and a patient's CT scan in different section views.

The slice shown in Figure 4A indicates an excellent agreement between these two, with estimated errors less than 20% of the stent width. More comparisons for different slices are shown in Figure 4B, where a visual examination shows good agreements in all of them.

### Final valve assembly

The last step is to apply the transformation functions obtained from the stent registration process to the original geometry files of the valve. Here, the STL format was used for the clean valve model. An STL files consists of vertices and faces (connections among the vertices). The transformations were applied to individual vertices, mapping them to their final locations. Since the connectivity of the vertices won't change during the deformation, a new mesh could be generated by using the mapped vertices and their original connectivity map. The transformations can be applied to the stent and also the leaflets and the skirt, and the final patient-specific clean valve model was obtained by assembling all the transformed components (Figure 5).

Figure 5 shows a sample patient-specific (self-expandable) valve model obtained using the method of Figure. 1.

One should note that the areas of the leaflets were not preserved during the process. As shown by the example in Figures 5A-5C, the patient's valve is bent to its side and appears oval. One last step is to use the metadata from the patient's DICOM file to transform the model from the image coordinate system to the patient's physical coordinate system. Post TAVR evaluations and ViV pre-surgical assessment can be carried out subsequently using this model.

Furthermore, since the orientation of the leaflet was also recovered during the process, it is now possible to evaluate the risk of coronary obstruction in the ViV simulation, which would be quite a difficult task before.

### The exemplary algorithm can be applied to other types of valves.

Figure 6A shows a balloon-expandable Edwards^{®} Sapien 3 valve model generated by reverse-engineering the micro-CT scans. The same procedures were applied, and the final results are compared in Figure 6B. Using 12 landmarks (6 on top and 6 at the bottom), the agreement between the stent model and the patient's geometry is perfect (Figure 6B). Because the stent design is axis-symmetric, matching the landmarks will not guarantee the correct leaflet orientation. The method may involve aligning at least one commissural point of the leaflets in the patient's CT scans to the commissure of the valve model. The final deformed patient specific valve model is shown in Figures 6C and 6D, after applying the transformations to the stent, leaflets, and skirt geometry. The valve is heavily deformed and has an oval cross-section as well.

### Error assessment

Although visual examinations have already shown good agreements between the registered valve and the ground truth, quantitative error evaluation using synthetic CT data was performed. The ground truth, a previously obtained deformed valve, was voxelized and applied with a blurring algorithm to mimic the blooming artifacts in CT scans. Then, the image registration-based process was carried out to reconstruct the stent geometry from this synthetic CT data. Lastly, the results were compared with the ground truth. An example is shown in Figure 7, a deformed stent is shown in Figure 7A. The geometry was converted into a 3D image array (voxelization) with a grid spacing of 0.35mm, the same resolution as the original CT scan. The blurring algorithm was a Gaussian filter with a kernel width of 9 pixels and a standard deviation of 1. The stent segmented from this synthetic CT data is shown in Figure 7B, where the width of the stent is measured at 1.5mm (4-5 pixels). The same 12 landmarks (same as in Section Multi-pass registration framework) were picked, and the whole process was repeated using a B-spline grid size of 63 to recover the stent geometry. The results are compared in Figure 7C, where the recovered stent is shaded. The two models look identical. Since both models have the same mesh (the same vertices and faces) and in the same coordinate system, it is possible to use the distances between corresponding vertices to quantify the error. The mean distance between the nodes of the two models is 0.106±0.091mm, which is less than one-third of a stent width of 0.45mm. The distribution of the errors is shown in Figure 7D, where 95% of the error is less than 0.29mm.

### Sensitivity analysis

The effects of the number of landmarks used, deformation grid size, and human input biases were evaluated using the same synthetic CT data.

Grid size: The sensitivity of this method to the grid size in the non-rigid registration step is shown in Figure 8A. The grid size has been varied from zero (rigid) to 23, 43, 63, and 83, and the same 12 landmarks were used for all grid sizes. The mean error plot was obtained from 10 different patient-specific valves, including 8 Evoluts and 2 Sapiens. For the case with only rigid registration, the mean error (0.80±0.32mm) was well above the width of the stent. However, it reduced dramatically to less than 0.2mm by the non-rigid B-spine registration with a 43 grid. The mean error kept decreasing to around 0.1mm when the grid size increased from 43 to 83. However, the computing time (on an eight-core desktop computer and only for the Evolut case shown in Figure 5) has increased drastically, from less than 10 minutes for the 23 grid to over 27 minutes for the 83 grid. The results have shown that the method works well at a reasonable computational cost when used with a moderately fine grid size (e.g., 43). Using very fine grids would not improve the results much, as resolutions of typical CT scans are usually above 0.3mm.

The number of landmarks used: The effects of the number of landmarks used in the registration process are shown in Figure 8B for a single Evolut case. Starting with 30 landmarks as the baseline, the number was reduced to 16, 12, 8, 6 and 4. The subsequent non-rigid registrations were all carried out on a 63 grid. The mean error only increased slightly with fewer landmarks used (Figure 8B), from 0.102mm for the baseline case to 0.112mm when only 4 landmarks were used. It indicates that this method is not sensitive to the number of landmarks. However, the computing time had increased with fewer landmarks, suggesting the algorithms took more time to find the perfect match when less initial information was given. After all, the results have shown that even with less than ten landmarks, the algorithm can produce accurate results reasonably fast.

Input biases: Lastly, to evaluate the sensitivity to human input biases, random numbers ranging from -1.5mm to 1.5mm (9 voxels in variations) were added to the three coordinates of all the landmarks, representing human input errors. Fifteen independent tests were carried out for the Evolut case with the same 12 landmarks and on a grid size of 63. The mean error from all the 15 tests was 0.146±0.017mm. Although it was higher than the case with correctly chosen landmarks, the registration error was still within 1/3 of a stent width.

### Leaflet deformation and stress calculation

Although the current method provides an estimation of the final leaflet geometry (Figures 5 and 6), correct deformation and residual stress distributions on the leaflets are crucial as the input for fluid-structure simulations of the flow. One way to obtain such information is to perform finite element analysis with the boundary conditions derived from the registration results. Specifically, between the original stent mesh and the deformed one after registration, the displacements of individual vertices can be calculated. With that information, the deformed stent can be recovered by applying the displacement conditions to the original stent in FEA simulations. Since the stress distribution in the stent is not the focus here, one can use any material models in this simulation. By applying tie constraints between the stent and the leaflet meshes, the leaflets will deform with the stent when the displacement boundary conditions are applied. The geometry and stress distributions on the leaflet can thus be correctly calculated. As an example, the deformed leaflets for the Evolut valve (same as in Figure 5) are shown in Figure 9 shaded by stress distributions (units are in MPa) on the bioprosthetic leaflets. The commissural lines between the leaflets are no longer straight, opposed to those shown in Figures 5B and 5C for the same model.

### Stent stress estimation

In this disclosure, a simple method to reasonably estimate the residual stress in self-expandable stents is illustrated.

To estimate the stress distribution in the recovered model, a crimping box method is introduced here. The crimping process and the comparisons of stress distributions are shown in Figure 10. Specifically, a bounding box of the stent was constructed and meshed with quad shell elements in Hypermesh. Since this bounding box (Figure 10A) had the exact shape of the undeformed stent, by applying the same transformations as the stent, it should match the shape of the deformed stent. Thus, instead of specifying displacements to the vertices of the stent, displacements were applied to the bounding box, using it to 'crimp' the valve model into its final shape (Figure 10B). The contact property between the box and the stent was defined using the 'hard' contact model with a friction coefficient of 0.1 in Abaqus. The stent material was the same superelastic Nitinol. A slight over-crimping followed by a releasing step was applied to account for the non-linear behavior of Nitinol. In this way, the local errors were damped out to avoid spikes in the stress distributions. The recovered stent and stress distributions (Figure 10C) are compared with the ground truth in Figure 10D. The recovered stent geometry has differences around 1mm, and the calculated stress distribution is slightly higher (~60MPa or 15% of the peak stress) when compared with the ground truth. Overall, this crimping method produces a mesh and stress distribution similar to the ground truth with minimal efforts compared with the process described by Gessat et al.

The exemplary method is not limited to TAVR, but can also be applied to recover the geometries of surgical bioprosthetic valves or any known implants from CT scans. With a database of patient-specific valve models generated by this method, one can train a deep learning model to fast recover the stent geometry.

It will be apparent to those skilled in the art that various modifications and variations can be made in the present disclosure without departing from the scope of the invention. Other aspects of the disclosure will be apparent to those skilled in the art from consideration of the specification and practice of the methods disclosed herein. It is intended that the specification and examples be considered as exemplary only, with a true scope of the invention being indicated by the following claims.

It should be appreciated that the logical operations described above can be implemented (1) as a sequence of computer implemented acts or program modules running on a computing system and/or (2) as interconnected machine logic circuits or circuit modules within the computing system. The implementation is a matter of choice dependent on the performance and other requirements of the computing system. Accordingly, the logical operations described herein are referred to variously as state operations, acts, or modules. These operations, acts and/or modules can be implemented in software, in firmware, in special purpose digital logic, in hardware, and any combination thereof. It should also be appreciated that more or fewer operations can be performed than shown in the figures and described herein. These operations can also be performed in a different order than those described herein.

Figure 11 shows an illustrative computer architecture for a computer system 200 capable of executing the software components that can use the output of the exemplary method described herein. The computer architecture shown in Figure 11 illustrates an example computer system configuration, and the computer 200 can be utilized to execute any aspects of the components and/or modules presented herein described as executing on the analysis system or any components in communication therewith.

In an aspect, the computing device 200 may comprise two or more computers in communication with each other that collaborate to perform a task. For example, but not by way of limitation, an application may be partitioned in such a way as to permit concurrent and/or parallel processing of the instructions of the application. Alternatively, the data processed by the application may be partitioned in such a way as to permit concurrent and/or parallel processing of different portions of a data set by the two or more computers. In an aspect, virtualization software may be employed by the computing device 200 to provide the functionality of a number of servers that is not directly bound to the number of computers in the computing device 200. For example, virtualization software may provide twenty virtual servers on four physical computers. In an aspect, the functionality disclosed above may be provided by executing the application and/or applications in a cloud computing environment. Cloud computing may comprise providing computing services via a network connection using dynamically scalable computing resources. Cloud computing may be supported, at least in part, by virtualization software. A cloud computing environment may be established by an enterprise and/or may be hired on an as-needed basis from a third-party provider. Some cloud computing environments may comprise cloud computing resources owned and operated by the enterprise as well as cloud computing resources hired and/or leased from a third-party provider.

In its most basic configuration, computing device 200 typically includes at least one processing unit 220 and system memory 230. Depending on the exact configuration and type of computing device, system memory 230 may be volatile (such as random-access memory (RAM)), non-volatile (such as read-only memory (ROM), flash memory, etc.), or some combination of the two.

This most basic configuration is illustrated in Figure 11 by dashed line 210. The processing unit 220 may be a standard programmable processor that performs arithmetic and logic operations necessary for operation of the computing device 200. While only one processing unit 220 is shown, multiple processors may be present. As used herein, processing unit and processor refers to a physical hardware device that executes encoded instructions for performing functions on inputs and creating outputs, including, for example, but not limited to, microprocessors (MCUs), microcontrollers, graphical processing units (GPUs), and application specific circuits (ASICs). Thus, while instructions may be discussed as executed by a processor, the instructions may be executed simultaneously, serially, or otherwise executed by one or multiple processors. The computing device 200 may also include a bus or other communication mechanism for communicating information among various components of the computing device 200.

Computing device 200 may have additional features/functionality. For example, computing device 200 may include additional storage such as removable storage 240 and non-removable storage 250 including, but not limited to, magnetic or optical disks or tapes.

Computing device 200 may also contain network connection(s) 280 that allow the device to communicate with other devices such as over the communication pathways described herein. The network connection(s) 280 may take the form of modems, modem banks, Ethernet cards, universal serial bus (USB) interface cards, serial interfaces, token ring cards, fiber distributed data interface (FDDI) cards, wireless local area network (WLAN) cards, radio transceiver cards such as code division multiple access (CDMA), global system for mobile communications (GSM), long-term evolution (LTE), worldwide interoperability for microwave access (WiMAX), and/or other air interface protocol radio transceiver cards, and other well-known network devices. Computing device 200 may also have input device(s) 270 such as keyboards, keypads, switches, dials, mice, track balls, touch screens, voice recognizers, card readers, paper tape readers, or other well-known input devices. Output device(s) 260 such as printers, video monitors, liquid crystal displays (LCDs), touch screen displays, displays, speakers, etc. may also be included. The additional devices may be connected to the bus in order to facilitate communication of data among the components of the computing device 200. All these devices are well known in the art and need not be discussed at length here.

The processing unit 220 may be configured to execute program code encoded in tangible, computer-readable media. Tangible, computer-readable media refers to any media that is capable of providing data that causes the computing device 200 (i.e., a machine) to operate in a particular fashion. Various computer-readable media may be utilized to provide instructions to the processing unit 220 for execution. Example tangible, computer-readable media may include, but is not limited to, volatile media, non-volatile media, removable media and non-removable media implemented in any method or technology for storage of information such as computer readable instructions, data structures, program modules or other data. System memory 230, removable storage 240, and non-removable storage 250 are all examples of tangible, computer storage media. Example tangible, computer-readable recording media include, but are not limited to, an integrated circuit (e.g., field-programmable gate array or application-specific IC), a hard disk, an optical disk, a magneto-optical disk, a floppy disk, a magnetic tape, a holographic storage medium, a solid-state device, RAM, ROM, electrically erasable program read-only memory (EEPROM), flash memory or other memory technology, CD-ROM, digital versatile disks (DVD) or other optical storage, magnetic cassettes, magnetic tape, magnetic disk storage or other magnetic storage devices.

In light of the above, it should be appreciated that many types of physical transformations take place in the computer architecture 200 in order to store and execute the software components presented herein. It also should be appreciated that the computer architecture 200 may include other types of computing devices, including hand-held computers, embedded computer systems, personal digital assistants, and other types of computing devices known to those skilled in the art. It is also contemplated that the computer architecture 200 may not include all of the components shown in Figure 11, may include other components that are not explicitly shown in Figure 11, or may utilize an architecture different than that shown in Figure 11.

In an example implementation, the processing unit 220 may execute program code stored in the system memory 230. For example, the bus may carry data to the system memory 230, from which the processing unit 220 receives and executes instructions. The data received by the system memory 230 may optionally be stored on the removable storage 240 or the non-removable storage 250 before or after execution by the processing unit 220.

It should be understood that the various techniques described herein may be implemented in connection with hardware or software or, where appropriate, with a combination thereof. Thus, the methods and apparatuses of the presently disclosed subject matter, or certain aspects or portions thereof, may take the form of program code (i.e., instructions) embodied in tangible media, such as floppy diskettes, CD-ROMs, hard drives, or any other machine-readable storage medium wherein, when the program code is loaded into and executed by a machine, such as a computing device, the machine becomes an apparatus for practicing the presently disclosed subject matter. In the case of program code execution on programmable computers, the computing device generally includes a processor, a storage medium readable by the processor (including volatile and non-volatile memory and/or storage elements), at least one input device, and at least one output device. One or more programs may implement or utilize the processes described in connection with the presently disclosed subject matter, e.g., through the use of an application programming interface (API), reusable controls, or the like. Such programs may be implemented in a high-level procedural or object-oriented programming language to communicate with a computer system. However, the program(s) can be implemented in assembly or machine language, if desired. In any case, the language may be a compiled or interpreted language and it may be combined with hardware implementations.

Moreover, the various components may be in communication via wireless and/or hardwire or other desirable and available communication means, systems and hardware. Moreover, various components and modules may be substituted with other modules or components that provide similar functions.

The computer architecture 200 includes software and/or hardware components and modules needed to enable the function of the modeling, simulation, and methods disclosed in the present disclosure. In some aspects, the computer architecture 200 may include artificial intelligence (A.I.) modules or algorithms and/or machine learning (M.L.) modules or algorithms (e.g., stored in the system memory 230, removable storage 240, non-removable storage 250, and/or a cloud database). The A.I. and/or M.L. modules/algorithms may improve the predictive power of the models, simulations, and/or methods disclosed in the present disclosure. For example, by using a deep learning, A.I., and/or M.L. model training including patient information and any relevant input data to the computational model, the predictive power of the computational model may be greatly enhanced. The A.I. and/or M.I. modules/algorithms also help improving sensitivity and specificity of the prediction as the database grows. In some aspects, the computer architecture 200 may include virtual reality (VR), augmented reality (AR) and/or mixed reality display(s), headset(s), glass(es), or any other suitable display device(s) as a part of the output device(s) 260 and/or the input device(s) 270. In some aspects, the display device(s) may be interactive to allow an user to select from options including with or without AR, with or without VR, or fused with real time clinical imaging to help clinician interact and make decisions.

Although example aspects of the present disclosure are explained in some instances in detail herein, it is to be understood that other aspects are contemplated. Accordingly, it is not intended that the present disclosure be limited in its scope to the details of construction and arrangement of components set forth in the following description or illustrated in the drawings. The present disclosure is capable of other aspects and of being practiced or carried out in various ways.

It must also be noted that, as used in the specification and the appended claims, the singular forms "a," "an" and "the" include plural referents unless the context clearly dictates otherwise. Ranges may be expressed herein as from "about" or "approximately" one particular value and/or to "about" or "approximately" another particular value. When such a range is expressed, other exemplary aspects include from the one particular value and/or to the other particular value.

By "comprising" or "containing" or "including" is meant that at least the name compound, element, particle, or method step is present in the composition or article or method, but does not exclude the presence of other compounds, materials, particles, method steps, even if the other such compounds, material, particles, method steps have the same function as what is named.

In describing example aspects, terminology will be resorted to for the sake of clarity. It is intended that each term contemplates its broadest meaning as understood by those skilled in the art and includes all technical equivalents that operate in a similar manner to accomplish a similar purpose. It is also to be understood that the mention of one or more steps of a method does not preclude the presence of additional method steps or intervening method steps between those steps expressly identified. Steps of a method may be performed in a different order than those described herein without departing from the scope of the present disclosure. Similarly, it is also to be understood that the mention of one or more components in a device or system does not preclude the presence of additional components or intervening components between those components expressly identified.

As discussed herein, a "subject" may be any applicable human, animal, or other organism, living or dead, or other biological or molecular structure or chemical environment, and may relate to particular components of the subject, for instance specific tissues or fluids of a subject (e.g., human tissue in a particular area of the body of a living subject), which may be in a particular location of the subject, referred to herein as an "area of interest" or a "region of interest."

It should be appreciated that as discussed herein, a subject may be a human or any animal. It should be appreciated that an animal may be a variety of any applicable type, including, but not limited thereto, mammal, veterinarian animal, livestock animal or pet type animal, etc. As an example, the animal may be a laboratory animal specifically selected to have certain characteristics similar to human (e.g., rat, dog, pig, monkey), etc. It should be appreciated that the subject may be any applicable human patient, for example.

The term "about," as used herein, means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. In one aspect, the term "about" means plus or minus 10% of the numerical value of the number with which it is being used. Therefore, about 50% means in the range of 45%-55%. Numerical ranges recited herein by endpoints include all numbers and fractions subsumed within that range (e.g., 1 to 5 includes 1, 1.5, 2, 2.75, 3, 3.90, 4, 4.24, and 5).

Similarly, numerical ranges recited herein by endpoints include subranges subsumed within that range (e.g., 1 to 5 includes 1-1.5, 1.5-2, 2-2.75, 2.75-3, 3-3.90, 3.90-4, 4-4.24, 4.24-5, 2-5, 3-5, 1-4, and 2-4). It is also to be understood that all numbers and fractions thereof are presumed to be modified by the term "about."

## Claims

1. A computer-readable medium (230, 240, 250) comprising instructions when executed causing a processor (220) to generate a model of an *in vivo* heart valve device implanted in a patient, by:
obtaining a computer-aided design (CAD) model of a pre-implantation heart valve device;
performing an imaging scan on the patient having an implanted heart valve device to obtain at least one patient specific landmark;
performing a stent registration process comprising deforming the CAD model to align it with the implanted heart valve device based on fitting of the at least one patient specific landmark;
applying transformation functions obtained from the stent registration process to an original geometry file of the CAD model to obtain a constructed patient-specific valve model; and
simulating a valve leaflet and skirt for the constructed patient-specific valve model by finite element analysis (FEA) with boundary conditions derived from the stent registration process.

2. The computer-readable medium (230, 240, 250) of claim 1, wherein the CAD model of the pre-implantation heart valve device is reconstructed from a micro-CT scan of the pre-implantation valve.

3. The computer-readable medium (230, 240, 250) of claim 1, wherein the heart valve device is a transcatheter aortic valve (TAV), a transcatheter mitral valve (TMV), a bioprosthetic surgical valve (SAV), a metal or radiopaque implant.

4. The computer-readable medium (230, 240, 250) of claim 1, wherein the constructed patient-specific valve model further comprises a model for patient-specific post-surgical evaluation and assessment.

5. The computer-readable medium (230, 240, 250) of claim 1, wherein the constructed patient-specific valve model further comprises a high-fidelity pre-surgical evaluation or modeling interventional pre-procedural planning necessary for heart valve device replacement or reconstruction.

6. The computer-readable medium (230, 240, 250) of claim 5, wherein the high-fidelity pre-surgical evaluation comprises developing a boundary condition in the Valve-in-Valve (ViV).

7. The computer-readable medium (230, 240, 250) of claim 1, wherein the imaging scan is a CT scan or an MRI scan.

8. The computer-readable medium (230, 240, 250) of claim 1, wherein no more than twelve patient specific landmarks are obtained from the imaging scan.

9. The computer-readable medium (230, 240, 250) of claim 1, wherein the stent registration process is performed based on a multi-pass registration framework.

10. A method of generating a patient-specific heart valve device model comprising:
obtaining a computer-aided design (CAD) model of a pre-implantation heart valve device;
performing an imaging scan on a patient having an implanted heart valve device to obtain at least one patient specific landmark;
performing a stent registration process comprising deforming the CAD model to align with the implanted heart valve device based on fitting of the at least one patient specific landmark;
applying transformation functions obtained from the stent registration process to an original geometry file of the CAD model to obtain a constructed patient-specific valve model; and
simulating a valve leaflet and skirt for the constructed patient-specific valve model by finite element analysis (FEA) with boundary conditions derived from the stent registration process.

11. The method of claim 10, wherein the CAD model of the pre-implantation heart valve device is reconstructed from a micro-CT scan of the pre-implantation valve, and wherein the heart valve device is a transcatheter aortic valve (TAV), a transcatheter mitral valve (TMV), a bioprosthetic surgical valve (SAV), a metal or radiopaque implant.

12. The method of claim 10, wherein the constructed patient-specific valve model further comprises a model for patient-specific post-surgical evaluation and assessment.

13. The method of claim 10, wherein the constructed patient-specific valve model further comprises a high-fidelity pre-surgical evaluation or modeling interventional pre-procedural planning necessary for heart valve device replacement or reconstruction, and wherein the high-fidelity pre-surgical evaluation comprises developing a boundary condition in the Valve-in-Valve (ViV).

14. The computer-readable medium (230, 240, 250) of claim 9, wherein the multiple pass registration framework comprises performing an initial pass of alignment and a subsequent intensity-based, non-rigid B-spline registration.

15. The method of claim 10, wherein the stent registration process is performed based on a multi-pass registration framework comprising performing an initial pass of alignment and a subsequent intensity-based, non-rigid B-spline registration.

## Patentansprüche

1. Computerlesbares Medium (230, 240, 250) umfassend Anweisungen, die, wenn sie von einem Prozessor (220) ausgeführt werden, den Prozessor veranlassen, ein Modell einer in-vivo-Herzklappenvorrichtung, die in einen Patienten implantiert ist, zu erzeugen durch:
Erhalten eines computerunterstützten Konstruktions- (CAD-) Modells einer Herzklappenvorrichtung vor der Implantation;
Durchführen eines Bildgebungsscans an dem Patienten, der eine implantierte Herzklappenvorrichtung aufweist, um mindestens einen patientenspezifischen Orientierungspunkt zu erhalten;
Durchführen eines Stentregistrierungsprozesses, umfassend das Verformen des CAD-Modells, um es mit der implantierten Herzklappenvorrichtung basierend auf einer Anpassung des mindestens einen patientenspezifischen Orientierungspunkts auszurichten;
Anwenden von aus dem Stentregistrierungsprozess erhaltenen Transformationsfunktionen auf eine ursprüngliche Geometriedatei des CAD-Modells, um ein konstruiertes patientenspezifisches Klappenmodell zu erhalten; und
Simulieren eines Klappensegels und einer Schürze für das konstruierte patientenspezifische Klappenmodell mittels Finite-Elemente-Analyse (FEA) mit aus dem Stentregistrierungsprozess abgeleiteten Randbedingungen.

2. Computerlesbares Medium (230, 240, 250) nach Anspruch 1, wobei das CAD-Modell der Herzklappenvorrichtung vor der Implantation aus einem Mikro-CT-Scan der Klappe vor der Implantation rekonstruiert wird.

3. Computerlesbares Medium (230, 240, 250) nach Anspruch 1, wobei die Herzklappenvorrichtung eine Transkatheter-Aortenklappe (TAV), eine Transkatheter-Mitralklappe (TMV), eine bioprothetische chirurgische Herzklappe (SAV), ein metallisches oder röntgendichtes Implantat ist.

4. Computerlesbares Medium (230, 240, 250) nach Anspruch 1, wobei das konstruierte patientenspezifische Klappenmodell weiter ein Modell für die patientenspezifische postoperative Bewertung und Beurteilung umfasst.

5. Computerlesbares Medium (230, 240, 250) nach Anspruch 1, wobei das konstruierte patientenspezifische Klappenmodell weiter eine hochgenaue präoperative Bewertung oder Modellieren einer interventionellen präprozeduralen Planung, die für den Ersatz oder die Rekonstruktion der Herzklappenvorrichtung erforderlich ist, umfasst.

6. Computerlesbares Medium (230, 240, 250) nach Anspruch 5, wobei die hochgenaue präoperative Bewertung das Entwickeln einer Randbedingung in dem Valve-in-Valve (ViV) umfasst.

7. Computerlesbares Medium (230, 240, 250) nach Anspruch 1, wobei der Bildgebungsscan ein CT-Scan oder ein MRT-Scan ist.

8. Computerlesbares Medium (230, 240, 250) nach Anspruch 1, wobei nicht mehr als zwölf patientenspezifische Orientierungspunkte aus dem Bildgebungsscan erhalten werden.

9. Computerlesbares Medium (230, 240, 250) nach Anspruch 1, wobei der Stentregistrierungsprozess basierend auf einer mehrstufigen Registrierungs-Grundstruktur durchgeführt wird.

10. Verfahren zum Erzeugen eines patientenspezifischen Modells einer Herzklappenvorrichtung umfassend:
Erhalten eines computerunterstützten Konstruktions- (CAD-) Modells einer Herzklappenvorrichtung vor der Implantation;
Durchführen eines Bildgebungsscans an einem Patienten, der eine implantierte Herzklappenvorrichtung aufweist, um mindestens einen patientenspezifischen Orientierungspunkt zu erhalten;
Durchführen eines Stentregistrierungsprozesses, umfassend das Verformen des CAD-Modells, um es mit der implantierten Herzklappenvorrichtung basierend auf einer Anpassung des mindestens einen patientenspezifischen Orientierungspunkts auszurichten;
Anwenden von aus dem Stentregistrierungsprozess erhaltenen Transformationsfunktionen auf eine ursprüngliche Geometriedatei des CAD-Modells, um ein konstruiertes patientenspezifisches Klappenmodell zu erhalten; und
Simulieren eines Klappensegels und einer Schürze für das konstruierte patientenspezifische Klappenmodell mittels Finite-Elemente-Analyse (FEA) mit aus dem Stentregistrierungsprozess abgeleiteten Randbedingungen.

11. Verfahren nach Anspruch 10, wobei das CAD-Modell der Herzklappenvorrichtung vor der Implantation aus einem Mikro-CT-Scan der Klappe vor der Implantation rekonstruiert wird, und wobei die Herzklappenvorrichtung eine Transkatheter-Aortenklappe (TAV), eine Transkatheter-Mitralklappe (TMV), eine bioprothetische chirurgische Herzklappe (SAV), ein metallisches oder röntgendichtes Implantat ist.

12. Verfahren nach Anspruch 10, wobei das konstruierte patientenspezifische Klappenmodell weiter ein Modell für die patientenspezifische postoperative Bewertung und Beurteilung umfasst.

13. Verfahren nach Anspruch 10, wobei das konstruierte patientenspezifische Klappenmodell weiter eine hochgenaue präoperative Bewertung oder Modellieren einer interventionellen präprozeduralen Planung, die für den Ersatz oder die Rekonstruktion der Herzklappenvorrichtung erforderlich ist, umfasst, und wobei die hochgenaue präoperative Bewertung das Entwickeln einer Randbedingung in dem Valve-in-Valve (ViV) umfasst.

14. Computerlesbares Medium (230, 240, 250) nach Anspruch 9, wobei die mehrstufige Registrierungs-Grundstruktur das Durchführen eines ersten Ausrichtungsdurchlaufs und einer nachfolgenden intensitätsbasierten, nichtstarren B-Spline-Registrierung umfasst.

15. Verfahren nach Anspruch 10, wobei der Stentregistrierungsprozess basierend auf einer mehrstufigen Registrierungs-Grundstruktur durchgeführt wird, umfassend das Durchführen eines ersten Ausrichtungsdurchlaufs und einer nachfolgenden intensitätsbasierten, nichtstarren B-Spline-Registrierung.

## Revendications

1. Support lisible par ordinateur (230, 240, 250) comprenant des instructions qui, lorsqu'elles sont mises en œuvre, amènent un processeur (220) à générer un modèle d'un dispositif de valve cardiaque in vivo implanté chez un patient, en :
obtenant un modèle de conception assistée par ordinateur (CAO) d'un dispositif de valve cardiaque pré-implantation ;
mettant en œuvre un examen d'imagerie sur le patient présentant un dispositif de valve cardiaque implanté afin d'obtenir au moins un point de repère spécifique au patient ;
mettant en œuvre un procédé de recalage du stent comprenant le fait de déformer le modèle de conception assistée par ordinateur (CAO) pour l'aligner sur le dispositif de valve cardiaque implanté sur la base de l'ajustement du au moins un repère spécifique au patient ;
appliquant des fonctions de transformation obtenues à partir du procédé de recalage du stent à un fichier de géométrie d'origine du modèle CAO afin d'obtenir un modèle de valve construit spécifique au patient ; et,
simulant une lame et une jupe de valve pour le modèle de valve construit spécifique au patient par analyse par éléments finis (FEA) avec des conditions aux limites dérivées du procédé de recalage du stent.

2. Support lisible par ordinateur (230, 240, 250) selon la revendication 1, dans lequel le modèle de conception assistée par ordinateur (CAO) du dispositif de valve cardiaque pré-implantation est reconstruit à partir d'un examen de microtomographie (micro-CT) de la valve pré-implantation.

3. Support lisible par ordinateur (230, 240, 250) selon la revendication 1, dans lequel le dispositif de valve cardiaque est une valve aortique transcathéter (TAV), une valve mitrale transcathéter (TMV), une valve chirurgicale bioprothétique (SAV), un implant métallique ou radio-opaque.

4. Support lisible par ordinateur (230, 240, 250) selon la revendication 1, dans lequel le modèle de valve construit spécifique à un patient comprend en outre un modèle pour l'évaluation et l'appréciation postopératoires spécifiques au patient.

5. Support lisible par ordinateur (230, 240, 250) selon la revendication 1, dans lequel le modèle de valve construit spécifique à un patient comprend en outre une évaluation préchirurgicale à haute fidélité ou une modélisation d'une planification interventionnelle pré-procédurale nécessaire au remplacement ou à la reconstruction du dispositif de valve cardiaque.

6. Support lisible par ordinateur (230, 240, 250) selon la revendication 5, dans lequel l'évaluation préchirurgicale à haute fidélité comprend le fait de développer une condition aux limites dans le cadre de la Valve-in-Valve (ViV).

7. Support lisible par ordinateur (230, 240, 250) selon la revendication 1, dans lequel l'examen d'imagerie est un examen CT ou un examen IRM.

8. Support lisible par ordinateur (230, 240, 250) selon la revendication 1, dans lequel pas plus de douze points de repère spécifiques au patient sont obtenus à partir de l'examen d'imagerie.

9. Support lisible par ordinateur (230, 240, 250) selon la revendication 1, dans lequel le procédé de recalage du stent est mis en œuvre sur la base d'un cadre de recalage multipasse.

10. Procédé de génération d'un modèle de dispositif de valve cardiaque spécifique à un patient comprenant le fait :
d'obtenir un modèle de conception assistée par ordinateur (CAO) d'un dispositif de valve cardiaque pré-implantation ;
de mettre en œuvre un examen d'imagerie sur un patient présentant un dispositif de valve cardiaque implanté afin d'obtenir au moins un point de repère spécifique au patient ;
de mettre en œuvre un procédé de recalage du stent comprenant le fait de déformer le modèle de conception assistée par ordinateur (CAO) pour l'aligner sur le dispositif de valve cardiaque implanté sur la base de l'ajustement du au moins un repère spécifique au patient ;
d'appliquer des fonctions de transformation obtenues à partir du procédé de recalage du stent à un fichier de géométrie d'origine du modèle CAO afin d'obtenir un modèle de valve construit spécifique au patient ; et,
de simuler une lame et une jupe de valve pour le modèle de valve construit spécifique au patient par analyse par éléments finis (FEA) avec des conditions aux limites dérivées du procédé de recalage du stent.

11. Procédé selon la revendication 10, dans lequel le modèle de conception assistée par ordinateur (CAO) du dispositif de valve cardiaque pré-implantation est reconstruit à partir d'un examen de microtomographie (micro-CT) de la valve pré-implantation, et dans lequel le dispositif de valve cardiaque est une valve aortique transcathéter (TAV), une valve mitrale transcathéter (TMV), une valve chirurgicale bioprothétique (SAV), un implant métallique ou radio-opaque.

12. Procédé selon la revendication 10, dans lequel le modèle de valve construit spécifique à un patient comprend en outre un modèle pour l'évaluation et l'appréciation postopératoires spécifiques au patient.

13. Procédé selon la revendication 10, dans lequel le modèle de valve construit spécifique à un patient comprend en outre une évaluation préchirurgicale à haute fidélité ou une modélisation d'une planification interventionnelle pré-procédurale nécessaire au remplacement ou à la reconstruction du dispositif de valve cardiaque, et dans lequel l'évaluation préchirurgicale à haute fidélité comprend le fait de développer une condition aux limites dans le cadre de la Valve-in-Valve (ViV).

14. Support lisible par ordinateur (230, 240, 250) selon la revendication 9, dans lequel le cadre de recalage multipasse comprend le fait d'effectuer une première passe d'alignement et un recalage ultérieur, non rigide, de type B-spline, basé sur l'intensité.

15. Procédé selon la revendication 10, dans lequel le procédé de recalage du stent est mis en œuvre sur la base d'un cadre de recalage multipasse comprenant le fait d'effectuer une première passe d'alignement et un recalage ultérieur, non rigide, de type B-spline, basé sur l'intensité.
